# NEUE EUROPÄISCHE PATENTSCHRIFT

(11) **EP 2 070 907 B2**
(45) Veröffentlichungstag und Bekanntmachung des Hinweises auf die Entscheidung über den Einspruch: **06.09.2023**
(45) Hinweis auf die Patenterteilung: 22.07.2015
(21) Anmeldenummer: 08020689.9
(22) Anmeldetag: 28.11.2008
(51) Int. Cl.: C07C 201/08, C07C 205/06

(54) **Verfahren zur Herstellung von Nitrobenzol durch adiabate Nitrierung**
Method for manufacturing Nitrobenzol through adiabatic nitration
Procédé de fabrication de nitrobenzole par nitrification adiabate

(30) Priorität: 11.12.2007 DE 102007059513
(43) Veröffentlichungstag der Anmeldung: 17.06.2009
(73) Patentinhaber: Covestro Deutschland AG, 51373 Leverkusen (DE)
(72) Erfinder: Rausch, Andreas Karl, Dr., 41564 Kaarst (DE); Knauf, Thomas, Dr., 41542 Dormagen (DE); Bolton, Jeffrey, Dr., 40489 Düsseldorf Einbrungen (DE); Racoes, Alexandre, 47805 Krefeld (DE)
(74) Vertreter: Levpat

(56) Entgegenhaltungen:
- EP-A- 0 156 199
- EP-A- 1 816 117
- EP-A1- 0 668 263
- EP-A1- 1 508 563
- EP-A2- 0 436 443
- EP-A2- 1 132 347
- EP-A2- 1 291 078
- DE-C2- 3 409 717
- US-A- 2 256 999
- US-A- 4 091 042
- US-A- 4 994 242
- US-A- 5 313 009
- US-A- 5 763 697
- US-A1- 2003 055 300
- US-B1- 6 288 289
- LYLE F. ALBRIGHT et al.: "Nitration, Recent Laboratory and Industrial Developments", ACS Symposium Series 623, 2 April 1995 (1995-04-02), - 7 April 1995 (1995-04-07), pages 222-232, Anaheim, CA, USA
- THOMAS L. GUGGENHEIM: "The Adiabatic Mononitrobenzene Process from the Bench Scale in 1974 to a Total World Capacity Approaching 10 Million MTPY in 2012", Chemistry, Process Design, and Safety for the Nitration Industry, vol. 1155, 2013, pages 1-11,
- KARL-HEINZ WEHDE et al.: "Löslichkeitsverhalten anorganischer Sulfate in Schwefelsäuren unterschiedlicher Konzentration", Chem.-Ing.-Tech., vol. 57, no. 4, 1985, pages 348-349,
- Jürgen Böcker, Spektroskopie, Vogel Verlag, 1. Auflage (1997), Seiten 234-235

## Beschreibung

Die Erfindung betrifft ein Verfahren zur kontinuierlichen Herstellung von Nitrobenzol durch adiabate Nitrierung von Benzol mit einem Gemisch von Schwefelsäure und Salpetersäure in einer Reaktionszone, bei dem die wässrige Phase enthaltend Schwefelsäure in der Reaktionszone einen Gehalt an schwerlösliche Metallsulfate bildenden Metallionen von weniger als 900 mg/l aufweist und dabei der Gehalt an schwerlösliche Metallsulfate bildenden Metallionen für Calcium unterhalb von 100 mg/l und für Eisen unterhalb von 300 mg/l ist, jeweils bezogen auf das Volumen der wässrige Phase enthaltend Schwefelsäure.

Die vorliegende Erfindung betrifft ein kontinuierliches Verfahren zur Herstellung von Nitrobenzol durch adiabate Nitrierung von Benzol durch ein Gemisch von Schwefel- und Salpetersäure (Mischsäure). Ein solches Verfahren wurde erstmals in US-A-225 69 99 beansprucht und ist in heutigen Ausführungsformen in US-A-409 10 42, US 531 30 09 und US-A-576 36 97 beschrieben.

Den beschriebenen adiabaten Verfahren ist gemein, dass die Ausgangsstoffe Benzol und Salpetersäure in einem großen Überschuss an Schwefelsäure zur Reaktion gebracht werden, der die frei werdende Reaktionswärme und das bei der Reaktion gebildete Wasser aufnimmt. Zur Durchführung der Reaktion werden Salpetersäure und Schwefelsäure zur sogenannten Mischsäure vermischt und in diese Benzol dosiert, das mit der Salpetersäure zu Wasser und im Wesentlichen Nitrobenzol reagiert. Die Temperatur der Reaktionsmischung und die Konzentrationen von Benzol, Salpetersäure und Schwefelsäure werden so gewählt, dass nach einer Reaktionszone eine im Wesentlichen salpetersäurefreie Mischung aus Benzol, Nitrobenzol, Schwefelsäure und Wasser erhalten wird. Die dazu erforderlichen Temperaturen liegen üblicherweise zwischen 70 und 145°C. Zur Erstellung der Mischsäure werden üblicherweise Salpetersäure einer Konzentration von 60 - 98 Gew.-% und Schwefelsäure einer Konzentration von 60 - 96 Gew.-% eingesetzt. Benzol wird mindestens in der bezogen auf die Salpetersäuremenge stöchiometrischen Menge eingesetzt, bevorzugt aber im 2 - 10%igen Überschuss im Vergleich zur stöchiometrisch benötigten Benzolmenge. In dem erfindungsgemäßen Verfahren werden dieses Verfahren und diese Parameter bevorzugt ebenfalls realisiert.

Die Reaktionszone, in der Benzol und Salpetersäure zur Reaktion gebracht werden, kann aus einer Anordnung von Rührkesseln, einem Schlaufenreaktor oder einem Rohrreaktor bestehen, wobei eine gute Durchmischung für die Reaktion förderlich ist. Bevorzugt wird daher ein Rohrreaktor eingesetzt, in dem mehrere Dispergierelemente über die Länge des Rohrreaktors verteilt angeordnet sind, die eine intensive Durchmischung von Benzol, Salpetersäure und Schwefelsäure und Wasser gewährleisten. Ein solcher Reaktor, sowie die Form einsetzbarer Dispergierelemente sind beispielsweise in US-A-499 42 42 und in US 2003/0055300 A1 beschrieben. In dem erfindungsgemäßen Verfahren werden dieses Verfahren und diese Parameter bevorzugt ebenfalls realisiert.

Das im Wesentlichen salpetersäurefreie Reaktionsgemisch, das nach der Reaktionszone erhalten wird, wird einem Phasentrennapparat zugeführt, in dem sich zwei Phasen bilden, wobei die erste als Rohnitrobenzol bezeichnet wird und im Wesentlichen aus Nitrobenzol, Benzol und einer im Nitrobenzol gelösten Menge an Schwefelsäure und Wasser besteht, und die zweite als Absäure bezeichnete im Wesentlichen aus Wasser, Schwefelsäure und in der Schwefelsäure gelöstem Nitrobenzol besteht.

Der Phasentrennapparat hat die bestimmungsgemäße Aufgabe die Phasen des Rohnitrobenzols und der Absäure vollständig zu trennen, so dass nur die physikalisch lösbaren Anteile der jeweils anderen Phase nicht abgetrennt werden können. Aufgrund dieses physikalisch lösbaren Anteils enthält das Rohnitrobenzol immer einen Anteil an Schwefelsäure und die Absäure immer einen Anteil an Rohnitrobenzol. In dem erfindungsgemäßen Verfahren werden dieses Verfahren und diese Parameter bevorzugt ebenfalls realisiert.

Das im Phasentrennapparat abgetrennte Rohnitrobenzol wird üblicherweise und bevorzugt auch in dem erfindungsgemäßen Verfahren einer Wäsche und einer destillativen Aufarbeitung unterzogen, wie beispielsweise in EP 181 61 17 A1 beschrieben.

Die im Phasentrennapparat abgetrennte Absäure wird üblicherweise und bevorzugt auch in dem erfindungsgemäßen Verfahren in einen Apparat zur Blitzverdampfung des Wassers eingebracht, in dem durch Anlegen eines Unterdrucks und unter Ausnutzung der hohen Temperatur der Absäure, die durch die adiabate Reaktionsführung erreicht wurde, Wasser aus der Absäure verdampft wird, so dass eine konzentrierte Schwefelsäure erhalten wird, deren Konzentration im Wesentlichen der Konzentration vor der Reaktionszone entspricht. Gemäß den bisherigen Ausführungsformen der adiabaten Benzolnitrierung, die auch in dem erfindungsgemäßen Verfahren bevorzugt genutzt wird, wird die durch Blitzverdampfung gewonnene Schwefelsäure (Kreislaufsäure) in einem Pufferbehälter gesammelt und vollständig in die Reaktionszone zurückgeführt. Durch die vollständige Rückführung der Schwefelsäure wird die Reaktionswärme am effektivsten genutzt. Durch Rückführung der Schwefelsäure entsteht ein Schwefelsäurekreislauf, der im Wesentlichen aus Reaktionszone, Phasentrennapparat, Verdampfer, Pufferbehälter und verbindenden Leitungen besteht.

Es ist bekannt, dass in der Schwefelsäure Metallionen vorhanden sein können, die zusammen mit Sulfat in Schwefelsäure schwerlösliche Metallsulfate bilden. Diese Metalle umfassen die Elemente Al, Ca, Cr, Mg, Mn, Fe, Co, Ni, Cu, Sr, Cd und Ba, insbesondere Ca und Fe, also Calcium und Eisen. Übersteigt die Konzentration dieser schwerlösliche Metallsulfate bildenden Metallionen die Löslichkeitsgrenze, so fallen Metallsulfate in Form eines Feststoffes in der Schwefelsäure aus und werden als Feststoffe mit der Schwefelsäure im Kreislauf mitgeführt, bis sie sich an einer Fläche oder einer Engstelle absetzen und ansammeln.

Es ist ebenfalls bekannt, dass die Löslichkeitsgrenze der schwerlösliche Metallsulfate bildenden Metallionen stark von der Temperatur der Lösung, also der Schwefelsäure, abhängt. So lösen sich in kalter Schwefelsäure weniger Metallionen als in heißer und demzufolge fallen in kalter Schwefelsäure oder an Stellen, an denen Schwefelsäure abgekühlt wird, wie es z.B. in Wärmetauschern der Fall ist, bevorzugt Metallsulfate als Feststoff an. Dieser Anfall von Feststoffen in Wärmetauschern ist als problematisch anzusehen, da er zu einer Belegung der Oberfläche des Wärmetauschers und damit zu einer Verschlechterung des Wärmeübergangskoeffizienten führen kann und auch durch die Verringerung des freien Querschnitts der Leitungen im Wärmetauscher die mögliche Durchflussmenge durch denselben begrenzt. Tabelle 1 zeigt die Löslichkeitsgrenzen für Calcium und Eisen für einige ausgewählte Temperaturen (Quelle: K.-H. Wehde: Untersuchungen zum Löslichkeitsverhalten anorganischer Sulfate und zur Wärmeübertragung bei der Aufkonzentrierung verunreinigter Schwefelsäure, Doktorarbeit, Universität Essen, 1984, S. 65 & S. 70):

**Tabelle 1: Löslichkeitsgrenzen von Calcium und Eisenionen in 70 Gew.%-iger Schwefelsäure**

| Temperatur | Calcium, Ca²⁺ [mg/l] | Eisen, Fe²⁺ [mg/l] |
|---|---|---|
| 20°C | 105 | 300 |
| 60°C | --- | 770 |
| 100°C | 230 | 1670 |
| 110°C | 260 | 2360 |

Dem beschriebenen Phänomen der ausfallenden Metallsulfate trägt der Stand der Technik Rechnung, in dem durch periodisches Spülen all jener Wärmetauscher, die Kreislaufsäure führen, die aus der konzentrierten Schwefelsäure kristallisierten Metallsulfate entfernt werden (DE 340 97 17 C2).

Es wurde nun gefunden, dass sich niedrige Gehalte an Metallionen in der aus der Nitrierung erhaltenen Schwefelsäure positiv auf die Aufkonzentrierung der Schwefelsäure auswirken. So werden bei der Blitzverdampfung (auch Flash-Verdampfung genannt, d.h. eine mit einer Entspannung verbundene Verdampfung) der schwefelsäurehaltigen Absäure, die nach Abtrennung der wässrigen Phase aus dem aus der Nitrierung von Benzol erhaltenen Reaktionsgemisch erhalten wird, höhere Schwefelsäurekonzentrationen in der gewonnenen aufkonzentrierten Schwefelsäure erreicht, wenn der Gehalt an Metallionen niedrig ist. Dies ist vermutlich auf die verbesserte Verdampfbarkeit des Wassers im Blitzverdampfer bei niedrigen Gehalten an Metallionen in der Absäure zurückzuführen. So wurde gefunden, dass bei der Blitzverdampfung unter ansonsten identischen Bedingungen (gleiche Temperatur der Absäure, gleicher Schwefelsäure-Gehalt der Absäure, gleicher Druck im Blitzverdampfer) eine konzentrierte Schwefelsäure mit einer um bis zu 0,25 % höheren Konzentration an H₂SO₄ erhalten wird, wenn eine Absäure mit niedrigen Gehalten an Metallionen von unter 900 mg/l eingesetzt wird (Beispiel 4 und 5).

Es wurde nun weiterhin gefunden, dass niedrigere Metallionenkonzentrationen auch zu einer Siedetemperaturerniedrigung der Schwefelsäure führen, wodurch sich ebenfalls ein niedriger Bedarf an Energie zur Aufkonzentrierung der Schwefelsäure ergibt.

Es wurde weiterhin jetzt beobachtet, dass die problematischen Ablagerungen von Metallsulfaten nicht nur in Wärmetauschern auftreten können, sondern auch an allen Stellen, an denen die Konzentration der schwerlösliche Metallsulfate bildenden Metallionen hoch genug und die Temperatur niedrig genug ist, um zu einer Feststoffbildung zu führen und gleichzeitig die Fließgeschwindigkeit der Schwefelsäure oder der Querschnitt der schwefelsäureführenden Leitungen niedrig genug ist, um eine für das Verfahren störende Ansammlung der Metallsulfate herbeizuführen.

Daher können Ablagerungen von Metallsulfaten nicht nur in Wärmetauschern beobachtet werden, sondern auch als Ablagerungen auf den Böden von Tanks, an Messstellen wie Standmessungen und an Dispergierelementen, die bestimmungsgemäß kleine Durchlassöffnungen aufweisen, wie sie z.B. in US-A-499 42 42 beschrieben sind. Ebenso können Ablagerungen von Metallsulfaten auch innerhalb der Blitzverdampfer auftreten, in denen bestimmungsgemäß die Schwefelsäure abgekühlt wird, während Wasser verdampft und die Konzentration der Säure erhöht wird. Des Weiteren können Ablagerungen von Metallsalzen auch in den der Reaktion nachfolgenden Aufarbeitungsschritten, wie zum Beispiel in der Abwasseraufarbeitung durch mitgeschleppte Metallsulfate entstehen. Um den störenden Einfluss dieser Ablagerungen zu verringern, ist gemäß des oben zitierten Stands der Technik eine periodische Reinigung der betroffenen Anlagenteile vorgesehen und notwendig. Diese Reinigung ist jedoch mit Stillständen in der Produktion und damit mit zusätzlichen Kosten verbunden.

Aufgabe der vorliegenden Erfindung war es daher, ein kostengünstiges und sicheres Verfahren zur Verfügung zu stellen, in dem die Ablagerung von Feststoffen und die damit verbundene Verstopfungsgefahr, die beispielsweise zum Ausfall von Messstellen führen kann, minimiert ist und mit dem gleichzeitig die anfallende aus der Nitrierung erhaltene Schwefelsäure mit niedrigem Energieverbrauch wieder aufkonzentriert werden kann.

Überraschend wurde gefunden, dass auf eine Reinigung von Wärmetauschern und schwefelsäureführenden Leitungen von ausgefallenen festen Metallsulfaten verzichtet werden kann, wenn bei der Nitrierung des Benzols durch eine schwefel- und salpetersäurehaltige Mischsäure die durch Blitzverdampfung von Wasser wiedererhaltene Schwefelsäure nicht vollständig als Kreislaufsäure in die Reaktionszone zurückgeführt wird, sondern teilweise ausgeschleust und durch frische, metallionenarme Schwefelsäure ersetzt wird.

Dabei wird die Schwefelsäure in einem Maß ausgeschleust und erneuert, dass die Konzentrationen an schwerlöslichen Metallsulfaten bildenden Metallionen in der wässrigen Phase enthaltend Schwefelsäure in der Reaktionszone unterhalb der Konzentrationen der Löslichkeitsgrenze bleiben, also unterhalb 100 mg/l für Calcium und unterhalb 300 mg/l für Eisen. Die Summe der Konzentrationen aller schwerlösliche Metallsulfate bildenden Metallionen bleibt kleiner als 900 mg/l - bezogen auf die wässrige Phase enthaltend Schwefelsäure in der Reaktionszone - und besonders bevorzugt kleiner als 500 mg/l.

Eine wässrige Phase enthaltend Schwefelsäure in der Reaktionszone, die weniger als 900 mg/l an schwerlösliche Metallsulfate bildenden Metallionen enthält und dabei eine Konzentration für Calcium unterhalb von 100 mg/l und für Eisen unterhalb von 300 mg/l aufweist, kann durch mehrere Ausführungsformen dieser Erfindung in einer Anlage zur Herstellung von Nitrobenzol durch adiabate Nitrierung von Benzol erreicht werden.

Die Erfindung betrifft ein Verfahren zur kontinuierlichen Herstellung von Nitrobenzol durch adiabate Nitrierung von Benzol mit einem Gemisch von Schwefelsäure und Salpetersäure, dadurch gekennzeichnet, dass das bei der Nitrierung erhaltene Reaktionsgemisch in eine wässrige und eine organische Phase getrennt wird, aus der wässrigen Phase anschließend Wasser verdampft wird, und die dabei erhaltene aufkonzentrierte Schwefelsäure teilweise als Kreislaufsäure in einem Maß in die Nitrierung zurückgeführt wird, und zusätzlich Schwefelsäure mit einer vergleichbaren oder größeren Konzentration als diejenige der Kreislaufsäure, nämlich einer Konzentration von größer als 65 Gew.-% und einem Gehalt an schwerlösliche Metallsulfate bildenden Metallionen von 100 mg/l oder weniger, in einem Maß in die Nitrierung eingeführt wird, dass die Summe der Konzentrationen der schwerlösliche Metallsulfate bildenden Metallionen in der Reaktionszone kleiner als 900 mg/l und dabei für Calcium unterhalb von 100 mg/l und für Eisen unterhalb von 300 mg/l ist, jeweils bezogen auf das Volumen der wässrige Phase enthaltend Schwefelsäure.

Erfindungsgemäß handelt es sich bei den schwerlöslichen, Metallsulfate bildenden Metallionen um Ca und Fe, also Calcium und Eisen, und bevorzugt ferner um Al, Cr, Mg, Mn, Co, Ni, Cu, Sr, Cd und Ba. Bevorzugt ist der Gehalt dieser Metallionen (Al, Ca, Cr, Mg, Mn, Fe, Co, Ni, Cu, Sr, Cd und Ba) in der Summe in der Reaktionszone kleiner als 800 mg/l, besonders bevorzugt kleiner als 450 mg/l, bezogen auf das Volumen der wässrige Phase enthaltend Schwefelsäure.

Bevorzugt wird das erfindungsgemäße Verfahren dabei wie folgt durchgeführt:

Die Ausgangsstoffe Benzol und Salpetersäure werden in einem großen Überschuss an Schwefelsäure zur Reaktion gebracht, der die frei werdende Reaktionswärme und das bei der Reaktion gebildete Wasser aufnimmt. Zur Durchführung der Reaktion werden Salpetersäure und Schwefelsäure zur sogenannten Mischsäure vermischt und in diese Benzol dosiert, das mit der Salpetersäure zu Wasser und im Wesentlichen Nitrobenzol reagiert. Die Temperatur der Reaktionsmischung und die Konzentrationen von Benzol, Salpetersäure und Schwefelsäure werden bevorzugt so gewählt, dass nach einer Reaktionszone, üblicherweise im Nitrierreaktor, eine im Wesentlichen salpetersäurefreie Mischung aus Benzol, Nitrobenzol, Schwefelsäure und Wasser erhalten wird. Die dazu erforderlichen Temperaturen liegen üblicherweise zwischen 70° und 145°C. Zur Erstellung der Mischsäure werden üblicherweise Salpetersäure einer Konzentration von 60 - 98 Gew.-% und Schwefelsäure einer Konzentration von 60 - 96 Gew.-% eingesetzt. Benzol wird bevorzugt mindestens in der bezogen auf die Salpetersäuremenge stöchiometrischen Menge eingesetzt, bevorzugt aber im 2 - 10%igen Überschuss im Vergleich zur stöchiometrisch benötigten Benzolmenge.

Die Reaktionszone, in der Benzol und Salpetersäure zur Reaktion gebracht werden, kann aus einer Anordnung von Rührkesseln, einem Schlaufenreaktor oder einem Rohrreaktor bestehen, wobei eine gute Durchmischung für die Reaktion förderlich ist. Bevorzugt wird daher ein Rohrreaktor eingesetzt, in dem mehrere Dispergierelemente über die Länge des Rohrreaktors verteilt angeordnet sind, die eine intensive Durchmischung von Benzol, Salpetersäure und Schwefelsäure und Wasser gewährleisten. Ein solcher Reaktor, sowie die Form einsetzbarer Dispergierelement sind beispielsweise in US 499 42 42 und in US 2003/0055300 A1 beschrieben.

Das im Wesentlichen salpetersäurefreie Reaktionsgemisch, das nach der Reaktionszone erhalten wird, wird einem Phasentrennapparat zugeführt, in dem sich zwei Phasen bilden, wobei die erste als Rohnitrobenzol bezeichnet wird und im Wesentlichen aus Nitrobenzol, Benzol und einer im Nitrobenzol gelösten Menge an Schwefelsäure und Wasser besteht, und die zweite als Absäure bezeichnete im Wesentlichen aus Wasser, Schwefelsäure und in der Schwefelsäure gelöstem Nitrobenzol besteht.

Der Phasentrennapparat hat die bestimmungsgemäße Aufgabe die Phasen des Rohnitrobenzols und der Absäure vollständig zu trennen, so dass nur die physikalisch lösbaren Anteile der jeweils anderen Phase nicht abgetrennt werden können. Aufgrund dieses physikalisch lösbaren Anteils enthält das Rohnitrobenzol immer einen Anteil an Schwefelsäure und die Absäure immer einen Anteil an Rohnitrobenzol.

Das im Phasentrennapparat abgetrennte Rohnitrobenzol wird anschließend bevorzugt einer Wäsche und / oder einer destillativen Aufarbeitung (zum Beispiel durch schnelle Verdampfung) unterzogen, wie beispielsweise in EP 181 61 17 A1 beschrieben.

Die im Phasentrennapparat abgetrennte Absäure wird dazu bevorzugt in einen Apparat zur schnellen Verdampfung (Blitzverdampfung) des Wassers eingebracht, in dem durch Anlegen eines Unterdrucks und unter Ausnutzung der hohen Temperatur der Absäure, die durch die adiabate Reaktionsführung erreicht wurde, Wasser aus der Absäure verdampft wird, so dass eine konzentrierte Schwefelsäure erhalten wird, deren Konzentration im Wesentlichen der Konzentration vor der Reaktionszone entspricht. Gemäß den bisherigen Ausführungsformen der adiabaten Benzolnitrierung, die auch in dem erfmdungsgemäßen Verfahren bevorzugt genutzt wird, wird die durch die Verdampfung gewonnene Schwefelsäure in einem Pufferbehälter gesammelt und vollständig in die Reaktionszone zurückgeführt. Die zurückgeführte Schwefelsäure wird auch als Kreislaufsäure bezeichnet. Durch die vollständige Rückführung der Schwefelsäure wird die Reaktionswärme am effektivsten genutzt. Durch Rückführung der Schwefelsäure entsteht ein Schwefelsäurekreislauf, der im Wesentlichen aus Reaktionszone, Phasentrennapparat, Verdampfer, Pufferbehälter und verbindenden Leitungen besteht.

Es hat sich überraschenderweise gezeigt, dass die Salpetersäure, die für die Nitrierung eingesetzt wird, die wesentliche Quelle für das Einbringen von Metallionen in die Produktionsanlagen zur Herstellung von Nitrobenzol durch adiabate Nitrierung von Benzol ist. Die Metallionen gelangen durch Korrosion in die Salpetersäure (z.B. Fe, Ni, Cr, Al), wobei die Korrosion in der Salpetersäureanlage selbst oder in dem Transportmedium (Pipeline, Schiff) auftreten kann oder werden durch das Wasser, das bei der Herstellung von Salpetersäure eingesetzt wird, eingebracht (z.B. Mg, Ca, Ba). Die Konzentration an Metallionen in der Kreislaufsäure, kann somit dadurch beschränkt werden, dass eine Salpetersäure hoher Qualität verwendet wird. Um einen Anstieg der Metallionenkonzentration in der Kreislaufsäure zu vermeiden, wird bevorzugt eine Salpetersäure eingesetzt, die weniger als 10 mg/l, bevorzugt weniger als 5 mg/l, ganz bevorzugt weniger als 1 mg/l an schwerlösliche Metallsulfate bildenden Metallionen enthält.

Um eine Ansammlung von Metallionen in der Kreislaufsäure und damit auch in der wässrigen Phase enthaltend Schwefelsäure in der Reaktionszone vollständig auszuschließen, müsste eine metallionenfreie Salpetersäure eingesetzt werden. Deren Herstellung und Transport sind nicht praktikabel, so dass weitere Maßnahmen zur Erreichung einer niedrigen Metallionenkonzentration in der Kreislaufsäure und in der wässrigen Phase enthaltend Schwefelsäure in der Reaktionszone vorteilhaft sind.

Es hat sich auch überraschenderweise gezeigt, dass die Metallionenkonzentration in der nach Phasentrennung und Aufkonzentration gewonnenen, aufkonzentrierten und zurückgeführten Schwefelsäure (Kreislaufsäure) und in der wässrigen Phase enthaltend Schwefelsäure in der Reaktionszone am wirkungsvollsten reguliert werden kann, wenn die Kreislaufsäure nicht wie im Stand der Technik beschrieben vollständig in die Reaktionszone zurückgeführt wird, sondern ein Teil der Kreislaufsäure ausgeschleust und durch eine Schwefelsäure vergleichbarer oder höherer Konzentration (> 65 Gew.-% H₂SO₄) und einem Gehalt an schwerlösliche Metallsulfate bildenden Metallionen von 100 mg/l oder weniger ersetzt wird. Dazu wird Kreislaufsäure an einer beliebigen Stelle des Schwefelsäurekreislaufes -bevorzugt wenigstens umfassend Reaktionszone, Phasentrennapparat, Verdampfer, Pufferbehälter und verbindende Leitungen - ausgeschleust und an einer anderen beliebigen Stelle des Schwefelsäurekreislaufes frische Schwefelsäure eingespeist, bis sich die erwünschte Konzentration an schwerlösliche Metallsulfate bildenden Metallionen eingestellt hat, die erfindungsgemäß unterhalb von 900 mg/l und dabei für Calcium unterhalb von 100 mg/l und für Eisen unterhalb von 300 mg/l in der wässrigen Phase enthaltend Schwefelsäure in der Reaktionszone liegt.

Bei der Einspeisung ist zu berücksichtigen, dass geringe Mengen an Kreislaufsäure dem Schwefelsäurekreislauf auch schon dadurch entzogen werden, dass sie in dem Rohnitrobenzol gelöst den Phasentrennapparat verlassen. Die im Rohnitrobenzol gelöste Menge an Schwefelsäure überschreitet bei den in Phasentrennapparaten üblicherweise vorliegenden Temperaturen von 120° bis 145°C im Allgemeinen nicht einen Wert von 0,25 Gew.-% des Rohnitrobenzols. Alle darüber hinaus mit dem Rohnitrobenzol ausgetragenen Mengen an Schwefelsäure liegen nicht gelöst, sondern als separate zweite Phase (Emulsion) im Rohnitrobenzol vor und sind als ausgeschleuste Mengen an Schwefelsäure im Sinne dieser Erfindung zu verstehen.

Das Ausschleusen von Kreislaufsäure und das Einspeisen von frischer Schwefelsäure hat sich nur dann als wirkungsvoll erwiesen, wenn die frische Schwefelsäure eine vergleichbare oder höhere Konzentration an H₂SO₄ als die Kreislaufsäure aufweist und wenn die frische Schwefelsäure deutlich weniger Metallionen aufweist als die ausgeschleuste Kreislaufsäure. Erfmdungsgemäß wird als Ersatz der ausgeschleusten Kreislaufsäure eine Schwefelsäure eingesetzt, deren Konzentration an H₂SO₄ größer als 65 Gew.-%, besonders bevorzugt größer 70 Gew.-% und ganz besonders bevorzugt größer 95 Gew.% ist, und die einen Gehalt an schwerlösliche Metallsulfate bildenden Metallionen von 100 mg/l oder weniger und bevorzugt von weniger als 50 mg/l aufweist.

Durch die Anwendung einer einzelnen der drei vorgenannten Maßnahmen oder bevorzugt durch deren Kombination wird gewährleistet, dass in der wässrigen Phase enthaltend Schwefelsäure in der Reaktionszone ein Gehalt an schwerlösliche Metallsulfate bildenden Metallionen von weniger als 900 mg/l vorliegt. Die Nutzung einer solchen wässrigen Phase bzw. Schwefelsäure in einem Verfahren zur Herstellung von Nitrobenzol durch adiabate Nitrierung bringt mehrere Vorteile mit sich:

Durch die niedrigen Metallionen-Konzentrationen fallen in der Reaktionszone und auch im Kreislauf keine schwerlöslichen Metallsulfate mehr aus. Dadurch entfällt die Reinigung von Wärmetauschern, Messstellen und Pufferbehältern, die üblicherweise mit Schwefelsäure einer Konzentration von 96 Gew.-% durchgeführt wird, teilweise mit dem Ausbau der verunreinigten Apparate verbunden ist und damit sehr zeitaufwändig und kostenintensiv ist. Das erfindungsgemäße Verfahren erhöht somit die Anlagenverfügbarkeit und senkt den Aufwand für die Instandhaltung.

Durch das Einhalten niedriger Metallionenkonzentrationen in der wässrigen Phase enthaltend Schwefelsäure in der Reaktionszone bzw. in der im Kreislauf geführten Schwefelsäure besteht nicht mehr die Gefahr, dass so viele Metallsulfate in Form von Feststoffen anfallen, dass sich Feststoffe in der Reaktionszone, das heißt in Düsen oder Dispergierelementen, die bestimmungsgemäß üblicherweise kleine Durchtrittsöffnungen aufweisen, ansammeln und dadurch die Durchmischung in der Reaktionszone so herabsetzen, dass die adiabate Nitrierung von Benzol unvollständig verläuft. Tritt dieser Fall ein, so muss die Kreislaufsäure vollständig erneuert werden, was mit erheblichem Arbeitsaufwand sowie hohen Lager-, Entsorgungs- und Wiederbeschaffungskosten verbunden ist. Durch die erfindungsgemäße kontinuierliche Ausschleusung und Erneuerung der Kreislaufsäure wird dieser Aufwand erheblich reduziert.

Durch das Einhalten niedriger Metallionenkonzentrationen in der wässrigen Phase enthaltend Schwefelsäure in der Reaktionszone bzw. in der im Kreislauf geführten Schwefelsäure weist die in die Blitzverdampfer zur Aufkonzentrierung eingebrachte Schwefelsäure keine erhöhten Wärmedurchgangskoeffizienten und keine erhöhte Siedetemperatur auf, so dass sich kein Mehrbedarf an Energie zur Aufkonzentrierung ergibt.

Durch das Einhalten niedriger Metallionenkonzentrationen in der wässrigen Phase enthaltend Schwefelsäure in der Reaktionszone bzw. in der im Kreislauf geführten Schwefelsäure enthält auch der Rohnitrobenzolstrom, der aus dem Phasentrennapparat gewonnen wird, weniger Metallionen, da die Schwefelsäure, die darin gelöst oder als zweite Phase vorliegt weniger Metallionen enthält. Dies eröffnet die Möglichkeit diesen Rohnitrobenzolstrom durch einen Wärmetauscher mit engen Durchtrittsöffnungen zu leiten ohne befürchten zu müssen, dass in diesem Wärmetauscher Metallsulfate ausfallen und die ihn verstopfen würden. So kann das Rohnitrobenzol, das den Phasentrennapparat verlässt und üblicherweise Temperaturen zwischen 120° und 145 °C aufweist dazu genutzt werden durch einen Wärmetauscher das Benzol oder die Salpetersäure, die in die Reaktionszone geführt werden, vorzuwärmen. Durch diese Maßnahme werden Kühlkosten für das Rohnitrobenzol sowie Heizkosten für die Kreislaufsäure eingespart.

Das erfindungsgemäße Verfahren ist durch eine Reihe von Merkmalen charakterisiert, die sich nicht im Stand der Technik finden. So wird bei der Herstellung von Nitrobenzol durch adiabate Nitrierung von Benzol eine höhere Anlagenverfügbarkeit, niedrigere Instandhaltungskosten und niedrigere Betriebskosten erreicht, wenn eine wässrigen Phase enthaltend Schwefelsäure in der Reaktionszone mit einem Gehalt an schwerlösliche Metallsulfate bildenden Metallionen von weniger als 900 mg/l, bevorzugt weniger als 500 mg/l verwendet wird, wobei der Gehalt für Calcium unterhalb von 100 mg/l und für Eisen unterhalb von 300 mg/l ist. Dadurch wird ein Rohnitrobenzol gewonnen, dessen Wärme in einem Wärmetauscher nutzbar gemacht werden kann um die Ströme der Ausgangsstoffe Benzol oder Salpetersäure vorzuwärmen ohne dabei befürchten zu müssen, dass in dem Wärmetauscher Metallsulfate ausfallen, die ihn verstopfen würden. Die niedrigen Metallionenkonzentrationen können erreicht werden, indem eine metallionenarme Salpetersäure eingesetzt wird und ein Teil der Kreislaufsäure kontinuierlich oder periodisch ausgeschleust und durch frische metallionenarme Schwefelsäure einer vergleichbaren oder höheren Konzentration ersetzt wird.

Sollten ausnahmsweise dennoch feste Metallsulfate an irgendeiner Stelle gebildet werden, so können diese mit einer Schwefelsäure einer Konzentration von größer 80 Gew.-%, bevorzugt größer 96 Gew.-% H₂SO₄ wieder aufgelöst werden.

### Beispiele

### Beispiel 1 (Vergleichsbeispiel)

Ein Verfahren zur Herstellung von Nitrobenzol durch adiabate Nitrierung wird mit einer Reaktionszone, die am Einlass ein Dispergierelement zur Durchmischung von Benzol und Mischsäure aufweist, betrieben, wobei in der Kreislaufsäure ein Metallgehalt von 46 mg/l an Calcium und 950 mg/l an Eisen mittels Atomabsorptionsspektrometrie nachgewiesen wurde. Das Nitrierverfahren lässt sich mit dieser Kreislaufsäure betreiben. Die wässrige Phase enthaltend Schwefelsäure in der Reaktionszone hat einen Gehalt an schwerlösliche Metallsulfate bildenden Metallionen, bezogen auf das Volumen der wässrige Phase enthaltend Schwefelsäure, von 950 mg/l.

Dann wird eine Salpetersäure zur Nitrierung eingesetzt, die 29 mg/l an Calcium und 5 mg/l an Eisen aufweist. Durch den Verbrauch dieser Salpetersäure während der Nitrierung des Benzols und der vollständigen Zurückführung der Kreislaufsäure in die Reaktionszone steigt die Konzentration der Metallionen in der Kreislaufsäure auf 180 mg/l an Calcium und 1200 mg/l an Eisen an und es kommt zum Ausfall erheblicher Mengen an Metallsulfaten am Dispergierelement am Einlass der Reaktionszone, wodurch sich der Druckverlust am Dispergierelement von 13,5 auf 14,5 bar erhöht und sich der mögliche Durchsatz an Schwefelsäure um 18 % verringert. Dadurch sinkt die Produktivität an Nitrobenzol um 16 %. Die Anlage muss abgefahren und die Reaktionszone aufwändig mit 96%iger Schwefelsäure gereinigt werden, nachdem die Reinigung mit Prozesskondensat, das im Wesentlichen aus Wasser besteht, erfolglos blieb.

### Beispiel 2 (Vergleichsbeispiel)

Ein Verfahren zur Herstellung von Nitrobenzol durch adiabate Nitrierung von Benzol mit Mischsäure wird mit einer Kreislaufsäure betrieben, die einen Metallgehalt von 70 mg/l an Calcium, 20 mg/l an Aluminium, 80 mg/l an Nickel, 120 mg/l an Chrom und 660 mg/l an Eisen, also in der Summe 950 mg/l an schwerlösliche Metallsulfate bildenden Metallionen aufweist. Weitere schwerlösliche Metallsulfate bildende Metallionen konnten mittels Atomabsorptionsspektrometrie nicht nachgewiesen werden. Das Nitrierverfahren lässt sich mit dieser Kreislaufsäure betreiben. Die wässrige Phase enthaltend Schwefelsäure in der Reaktionszone hat einen Gehalt an schwerlösliche Metallsulfate bildenden Metallionen, bezogen auf das Volumen der wässrige Phase enthaltend Schwefelsäure, von 905 mg/l.

Das Rohnitrobenzol, das in dem Phasentrennapparat erhalten wird, wird in einem Plattenwärmetauscher zur Vorerwärmung des Benzols genutzt, während die Kreislaufschwefelsäure vollständig in die Reaktionszone zurückgeführt wird. Während eines Zeitraumes von 6 Wochen wird zur Nitrierung eine Salpetersäure eingesetzt, deren Gehalt an schwerlösliche Metallsulfate bildenden Metallionen in der Summe kleiner ist als 10 mg/l. Die wässrige Phase enthaltend Schwefelsäure in der Reaktionszone hat nach 6 Wochen einen Gehalt an schwerlösliche Metallsulfate bildenden Metallionen, bezogen auf das Volumen der wässrige Phase enthaltend Schwefelsäure, von 675 mg/l.

Nach 6 Wochen sind die Durchtrittsöffnungen des Plattenwärmetauschers durch ausgefallene Metallsulfate teilweise verstopft, wodurch die produzierte Menge an Rohnitrobenzol nicht vollständig abfließen kann und in den Phasentrennapparat zurück staut, wodurch die Phasentrennung gestört wird. Der Plattenwärmetauscher muss ausgebaut und gereinigt werden.

### Beispiel 3 (Erfindungsgemäßes Beispiel)

Ein Verfahren zur Herstellung von Nitrobenzol durch adiabate Nitrierung von Benzol mit Mischsäure wird mit einer Kreislaufsäure betrieben, die einen Metallgehalt von 90 mg/l an Calcium, 10 mg/l an Aluminium, 10 mg/l an Nickel, 10 mg/l an Chrom und 100 mg/l an Eisen, also in der Summe 220 mg/l an schwerlösliche Metallsulfate bildenden Metallionen aufweist. Weitere schwerlösliche Metallsulfate bildende Metallionen konnten mittels Atomabsorptionsspektrometrie nicht nachgewiesen werden. Das Nitrierverfahren lässt sich mit dieser Kreislaufsäure betreiben. Die wässrige Phase enthaltend Schwefelsäure in der Reaktionszone hat einen Gehalt an schwerlösliche Metallsulfate bildenden Metallionen, bezogen auf das Volumen der wässrige Phase enthaltend Schwefelsäure, von 200 mg/l.

Das Rohnitrobenzol, das in dem Phasentrennapparat erhalten wird, wird in einem Plattenwärmetauscher zur Vorerwärmung des Benzols genutzt.

Während eines Zeitraumes von 12 Wochen wird täglich eine Menge von 0,9 m³ Kreislaufsäure kontinuierlich ausgeschleust und durch 0,7 m³ einer Schwefelsäure mit einem Gehalt von 96 Gew.-% H₂SO₄ ersetzt. Der durchschnittliche Gehalt an schwerlösliche Metallsulfate bildenden Metallionen Fe, Cr, Ni, Al und Ca betrug über diesen Zeitraum für die zur Erneuerung eingesetzte 96 Gew.%ige Schwefelsäure in der Summe 50 mg/l und für die Salpetersäure 1 mg/l. Nach dem Zeitraum von 12 Wochen wurden keine Ablagerungen im Wärmetauscher und keine Veränderung des möglichen Durchsatzes durch die Reaktionszone festgestellt. Die wässrige Phase enthaltend Schwefelsäure in der Reaktionszone hat nach 6 Wochen einen Gehalt an schwerlösliche Metallsulfate bildenden Metallionen, bezogen auf das Volumen der wässrige Phase enthaltend Schwefelsäure, von 400 mg/l.

### Referenzbeispiel 4

Ein Verfahren zur Herstellung von Nitrobenzol durch adiabate Nitrierung von Benzol mit Mischsäure wird in einem zweistufigen Versuch mit einer Kreislaufsäure betrieben, die zunächst im ersten (offenbarten) Versuchsdurchgang einen Metallgehalt von 600 mg/l an schwerlösliche Metallsulfate bildenden Metallionen aufweist und schließlich im zweiten (nicht erfmdungsgemäßen) Versuchsdurchgang einen Metallgehalt von 1350 mg/l an schwerlösliche Metallsulfate bildenden Metallionen aufweist.

Die durch den Blitzverdampfer zu verdampfende Menge an Wasser beträgt ca. 10 Tonnen pro Stunde. Temperatur und Druck im Blitzverdampfer sind in den beiden Versuchsdurchgängen identisch und für den technischen Maßstab konstant (d.h., die maximale Schwankung beträgt ± 0,5°C und ± 1 mbar). Dennoch wird während der ersten Versuchsdurchgangs eine Schwefelsäure erhalten, die eine um 0,25 Gew.-% höhere Konzentration an H₂SO₄ aufweist als die Schwefelsäure, die während des zweiten Versuchsdurchgangs erhalten wird.

### Referenzbeispiel 5

Der Versuch in zwei Durchgängen gemäß Beispiel 4 wird wiederholt. Diesmal beträgt der Gehalt an schwerlösliche Metallsulfate bildenden Metallionen während des ersten (offenbarten) Versuchsdurchgangs 400 mg/l und während des zweiten (nicht erfindungsgemäßen) Versuchsdurchgangs 1900 mg/l. Die durch den Blitzverdampfer zu verdampfende Menge an Wasser beträgt ca. 8 Tonnen pro Stunde. Temperatur und Druck im Blitzverdampfer sind in den beiden Versuchsdurchgängen identisch und für den technischen Maßstab konstant (d.h., die maximale Schwankung beträgt ± 0,5°C und ± 1 mbar). Während des ersten Versuchsdurchgangs wird eine Schwefelsäure erhalten, die eine um 0,10 Gew.-% höhere Konzentration an H₂SO₄ aufweist als die Schwefelsäure, die während des zweiten Versuchsdurchgangs erhalten wird.

## Patentansprüche

1. Verfahren zur kontinuierlichen Herstellung von Nitrobenzol durch adiabate Nitrierung von Benzol mit einem Gemisch von Schwefelsäure und Salpetersäure, **dadurch gekennzeichnet, dass** das bei der Nitrierung erhaltene Reaktionsgemisch in eine wässrige und eine organische Phase getrennt wird, aus der wässrigen Phase anschließend Wasser verdampft wird,
und die dabei erhaltene aufkonzentrierte Schwefelsäure teilweise als Kreislaufsäure in einem Maß in die Nitrierung zurückgeführt wird,
und zusätzlich Schwefelsäure mit einer vergleichbaren oder größeren Konzentration als diejenige der Kreislaufsäure, nämlich einer Konzentration von größer 65 Gew.-% und einem Gehalt an schwerlösliche Metallsulfate bildenden Metallionen von 100 mg/l oder weniger, in einem Maß in die Nitrierung eingeführt wird,
dass die Summe der Konzentrationen der schwerlösliche Metallsulfate bildenden Metallionen in der Reaktionszone kleiner als 900 mg/l und dabei für Calcium unterhalb von 100 mg/l und für Eisen unterhalb von 300mg/l ist, jeweils bezogen auf das Volumen der wäss-rige Phase enthaltend Schwefelsäure.

2. Verfahren nach Anspruch 1, **dadurch gekennzeichnet, dass** die Reaktionszone zur adiabaten Nitrierung von Benzol mindestens zwei Dispergierelemente aufweist.

3. Verfahren nach Anspruch 1 oder 2, **dadurch gekennzeichnet, dass** die zur Nitrierung eingesetzte Salpetersäure weniger als 10 mg/l an schwerlösliche Metallsulfate bildenden Metallionen enthält.

4. Verfahren nach Anspruch 1, **dadurch gekennzeichnet, dass** die in der Phasentrennung erhaltene organische Phase zur Erwärmung des der Reaktionszone zugeführten Benzols genutzt wird.

5. Verfahren nach einem der Ansprüche 1 bis 4, **dadurch gekennzeichnet, dass** die schwerlöslichen, Metallsulfate bildenden Metallionen Al, Ca, Cr, Mg, Mn, Fe, Co, Ni, Cu, Sr, Cd und / oder Ba sind und der Gehalt dieser Metallionen in der Summe in der Reaktionszone kleiner als 800 mg/l, bezogen auf das Volumen der wässrige Phase enthaltend Schwefelsäure, ist.

## Claims

1. Process for the continuous preparation of nitrobenzene by adiabatic nitration of benzene by means of a mixture of sulfuric acid and nitric acid, **characterized in that** the reaction mixture obtained in the nitration is separated into an aqueous phase and an organic phase, water is subsequently evaporated from the aqueous phase,
and the concentrated-up sulfuric acid obtained is partly recirculated to the nitration as circulated acid,
and sulfuric acid having a comparable or greater concentration than that of the circulated acid, namely a concentration of greater than 65% by weight, and a content of metal ions which form sparingly soluble metal sulfates of 100 mg/l or less is additionally introduced into the nitration,
in such amounts that the sum of the concentrations of the metal ions which form sparingly soluble metal sulfates in the reaction zone is less than 900 mg/l, and at the same time is below 100 mg/l for calcium and below 300 mg/l for iron, based in each case on the volume of the aqueous phase containing sulfuric acid.

2. Process according to Claim 1, **characterized in that** the reaction zone for the adiabatic nitration of benzene has at least two dispersing elements.

3. Process according to Claim 1 or 2, **characterized in that** the nitric acid used for the nitration contains less than 10 mg/l of metal ions which form sparingly soluble metal sulfates.

4. Process according to Claim 1, **characterized in that** the organic phase obtained in the phase separation is used to heat the benzene fed into the reaction zone.

5. Process according to any of Claims 1 to 4, **characterized in that** the metal ions which form sparingly soluble metal sulfates are Al, Ca, Cr, Mg, Mn, Fe, Co, Ni, Cu, Sr, Cd and/or Ba and the total content of these metal ions in the reaction zone is less than 800 mg/l, based on the volume of the aqueous phase containing sulfuric acid.

## Revendications

1. Procédé de préparation continue de nitrobenzène par nitration adiabatique de benzène avec un mélange d'acide sulfurique et d'acide nitrique, **caractérisé en ce que** le mélange réactionnel obtenu lors de la nitration est séparé en une phase aqueuse et une phase organique, de l'eau étant ensuite évaporée de la phase aqueuse,
et l'acide sulfurique concentré ainsi obtenu est renvoyé partiellement sous forme d'acide recyclé en une quantité dans la nitration,
et en outre de l'acide sulfurique, ayant une concentration comparable ou supérieure à celle de l'acide recyclé, plus précisément une concentration supérieure à 65 % en poids, et ayant une teneur en ions métalliques formant des sulfates métalliques difficilement solubles de 100 mg/l ou moins, est introduit en une quantité dans la nitration,
telles que la somme des concentrations des ions métalliques formant des sulfates métalliques difficilement solubles dans la zone de réaction soit inférieure à 900 mg/l, et alors, pour le calcium, inférieure à 100 mg/l et pour le fer, inférieure à 300 mg/l, dans chaque cas par rapport au volume de la phase aqueuse contenant l'acide sulfurique.

2. Procédé selon la revendication 1, **caractérisé en ce que** la zone de réaction destinée à la nitration adiabatique du benzène comprend au moins deux éléments de dispersion.

3. Procédé selon la revendication 1 ou 2, **caractérisé en ce que** l'acide nitrique utilisé pour la nitration contient moins de 10 mg/l d'ions métalliques formant des sulfates métalliques difficilement solubles.

4. Procédé selon la revendication 1, **caractérisé en ce que** la phase organique obtenue lors de la séparation des phases est utilisée pour chauffer le benzène envoyé dans la zone de réaction.

5. Procédé selon l'une des revendications 1 à 4, **caractérisé en ce que** les ions métalliques formant des sulfates métalliques difficilement solubles sont Al, Ca, Cr, Mg, Mn, Fe, Co, Ni, Cu, Sr, Cd et/ou Ba, et la teneur totale en ces ions métalliques de la zone de réaction est inférieure à 800 mg/l, par rapport au volume de la phase aqueuse contenant l'acide sulfurique.
